(19) Europäisches Patentamt
European Patent Office
Office européen des brevets

(11) Publication number: **0 418 076 A2**

# (12) EUROPEAN PATENT APPLICATION

(21) Application number: 90310030.3

(22) Date of filing: 13.09.90

(51) Int. Cl.[5]: C07H 21/00, C12Q 1/68

(30) Priority: 14.09.89 JP 237329/89

(43) Date of publication of application: 20.03.91 Bulletin 91/12

(84) Designated Contracting States: BE DE FR GB IT NL

(71) Applicant: TOSOH CORPORATION
4560, Oaza-Tonda Shinnanyo-shi
Yamaguchi 746(JP)

(72) Inventor: Yamagishi, Hiroaki
2-7-3 Tachibanadai, Midori-ku
Yokohama-shi, Kanagawa-ken 227(JP)
Inventor: Mitoma, Yasutami 106, Sun Palace Shonan
26-5, Shonandai 4-chome, Fujisawa-shi,
Kanagawa 252(JP)

(74) Representative: Kearney, Kevin David Nicholas et al
KILBURN & STRODE 30 John Street
London, WC1N 2DD(GB)

## (54) Oligonucleotide derivative and process for producing the same.

(57) Novel oligonucleotide derivatives useful as ligands or DNA probes to be immobilized in a carrier are disclosed. The oligonucleotide derivatives of the present invention are represented by the formula [I] or [II].

(5′) 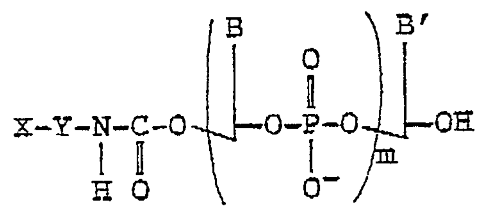 (3′) [I]

(wherein m represents a natural number, B and B′, the same or different, represent a base selected from the group consisting of adenine, thymine, cytosine, and guanine,

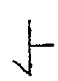

represents a deoxyribose residue which lacks hydroxyl groups on 3′ and 5′ position of 2′-deoxyribose, X represents a functional group which can react with other materials, and Y represents a linker).

(5′) 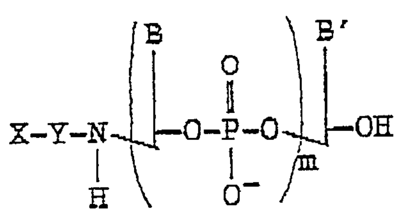 (3′) [II]

EP 0 418 076 A2

(wherein

m, B, B', X, 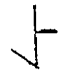, and Y

represent the same meanings as in formula [I]).

## BACKGROUND OF THE INVENTION

### I. Field of the Invention

This invention relates to an oligonucleotide derivative and a processe for producing the same. The oligonucleotide derivative of the present invention is useful as a ligand for affinity chromatography or a DNA probe, which is used in the immobilized state.

### II. Description of the Related Art

Nucleic acids are hitherto used in various fields. For example, a useful protein can be artificially produced by using a nucleic acid encoding the amino acid sequence of the protein. By using a synthetic nucleic acid, diagnosis or probing of a gene may be attained. Further, by using a nucleic acid immobilized on a carrier, a mRNA or the like may be separated and purified. Further, nucleic acids may be used as primers for the determinations of base sequences of nucleic acids (e.g., by Sanger's method).

In the above-mentioned various uses of the nucleic acids, in cases where the nucleic acid is to be used in a state immobilized on a carrier, the amino groups in the nucleic acid and the epoxy groups in the carrier are conventionally reacted. However, by this method, the amount of the nucleic which is fixed to the carrier is small. Further, since the nucleic acid may be fixed to the carrier at two or more points, the mobility of the nucleic acid may be degraded very much.

In cases where a nucleic acid is used as a probe, the nucleic acid is usually labelled with a marker such as a radio isotope, enzyme or a fluorescent substance. The labelled nucleic acid is conventionally prepared by attaching preliminarily labelled nucleotide monomers to the 3′ end of a DNA using terminal transferase or by nick translation method. However, these methods cost expensive because special enzymes are used. Further, since enzyme reactions are utilized, the operation is complicated and time-consuming. Further, in the latter method, it is difficult to synthesize nucleic acid probes having a uniform and arbitrary base sequence.

## SUMMARY OF THE INVENTION

Accordingly, an object of the present invention is to provide a nucleic acid derivative which may be fixed to a carrier without degrading the mobility of the nucleic acid so much.

Another object of the present invention is to provide a nucleic acid derivative which may be labelled with a marker by a simple and inexpensive method.

Still another object of the present invention is to provide a process for producing the oligonucleotide derivative of the present invention.

To attain these and other objects of the present invention, there is provided an oligonucleotide derivative represented by the formula [I]:

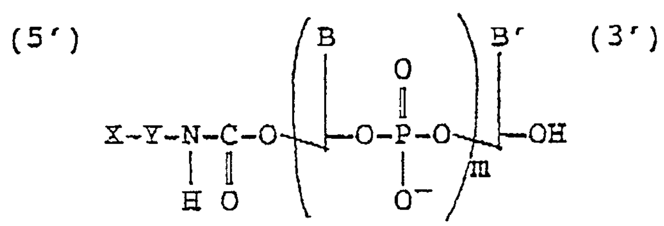

[I]

(wherein m represents a natural number, B and B′, the same or different, represent a base selected from the group consisting of adenine, thymine, cytosine and guanine,

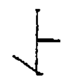

represents a deoxyribose residue which lacks hydroxyl groups on 3′ and 5′ position of 2′-deoxyribose, X represents a functional group which can react with other materials, and Y represents a linker).

The present invention also provides an oligonucleotide derivative represented by the formula [II]:

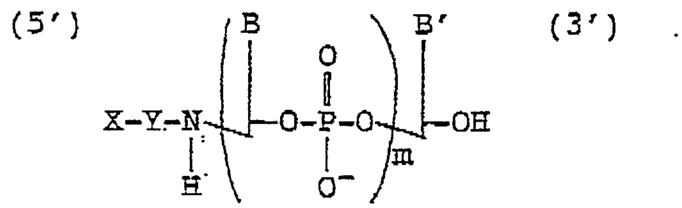

[II]

(wherein m, B, B′, X,

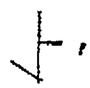

and Y represent the same meanings as in formula [I]).

The present invention further provides a process of producing the oligonucleotide derivative represented by the formula [I] comprising the steps of: reacting an oligonucleotide derivative represented by the formula [III]:

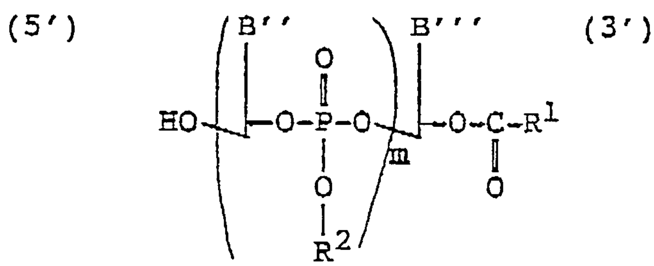

[III]

(wherein $-COR^1$ represents a protection group of the hydroxyl group at the 3′ end of the oligonucleotide derivative, $R^2$ represents a protection group of the phosphate group, m and

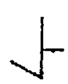

represent the same meaning as in formula [I], B″ and B‴, the same or different, represent adenine, cytosine, guanine or thymine of which amino group may be protected)
with N,N′-carbonyldiimidazole to form an active intermediate represented by the formula [IV]:

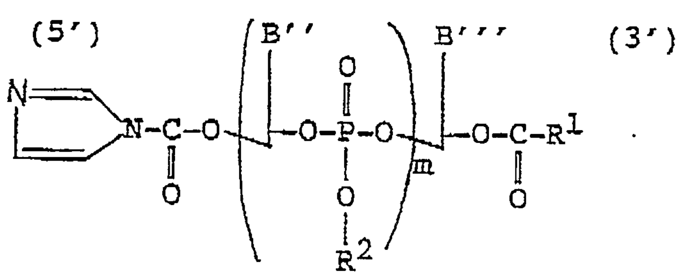

[IV]

(wherein m, B′, B‴,

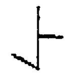

, -COR¹ and R² represent the same meanings as in formula [III]); reacting the obtained active intermediate with a compound represented by the formula $NH_2$-Y-X (wherein Y and X represent the same meanings as in formula (I)]; and removing said protection groups represented by -COR¹ and -R².

The present invention still further provides a process of producing the oligonucleotide derivative represented by the formula [II], comprising the steps of: reacting said oligonucleotide derivative represented by said formula [III] with a 2-fluoro-1-methylpyridinium salt to form an active intermediate represented by the formula [V]:

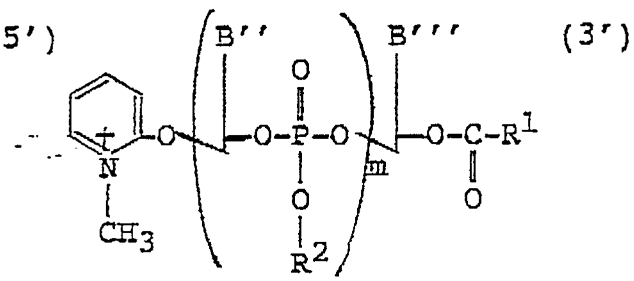

[V]

(wherein m, B′, B‴,

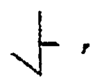

R¹ and R² represent the same meanings as in formula [III]); reacting the obtained active intermediate with a compound represented by the formula $NH_2$-Y-X (wherein Y and X represent the same meanings as in formula [I]); and removing said protection groups represented by -COR¹ and -R².

The oligonucleotide derivative of the present invention has a functional group such as primary amino group, which has higher reactivity than the hydroxyl groups or phosphate groups existing in the nucleotide molecule or than amino groups existing in the bases of the nucleotide. Thus, the oligonucleotide derivative of the present invention can be easily and selectively reacted with other substances such as carriers and markers so as to form a linkage between the functional group of the oligonucleotide derivative and a group in the other substance. The oligonucleotide derivative of the present invention may be designed so as to have a uniform and arbitrary base sequence. Thus, by the present invention, oligonucleotide derivative may appropriately be used for providing an immobilized oligonucleotide for affinity chromatography or as a hybridization probe.

The process for preparing the oligonucleotide derivative of the present invention is simpler, less expensive and less time-consuming than the conventional methods utilizing an enzyme. Further, unlike the conventional methods, since it is not necessary to prepare nucleotide monomers separately, the production process may be automated.

## BRIEF DESCRIPTION OF THE DRAWINGS

Fig. 1 shows a chromatogram of ion-exchange chromatography of the oligonucleotide derivative of the present invention and the oligonucleotide derivative having a hydroxyl group at its 5′ end, which is a starting material of the oligonucleotide derivative of the present invention; and

Fig. 2 shows reverse phase chromatogram of the oligonucleotide derivative of the present invention labelled with fluorescein.

DETAILED DESCRIPTION OF THE PREFERRED EMBODIMENTS

As described above, the oligonucleotide derivative of the present invention is represented by the formula [I] or [II].

In these formulae, m represents a natural number. Although the number of m is not restricted, it is usually in the range of 5 to 100.

In the formulae, B and $B'$ represent a base constituting the naturally occurring nucleotides, that is, adenine, thymine, cytosine or guanine. Needless to say, the bases in different recurring units may be the same or different. More particularly, the sequence of the bases may be designed so as to provide a desired ligand to be used in affinity chromatography or a DNA probe.

In the formulae,

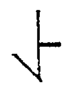

represents a deoxyribose residue which lacks hydroxyl groups on $3'$ and $5'$ position of $2'$-deoxyribose. More particularly,

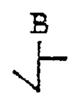

means a deoxyribose residue represented by the formula

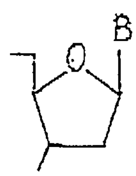

In the formulae [I] and [II], X represents a functional group which can react with other substances. Preferred examples of the functional group X may include primary and secondary amino groups, carboxylic group, thiol group and hydroxyl group. The functional group X may be selected so that the oligonucleotide derivative can easily and/or stably react with the functional group of a marker or a carrier to which the oligonucleotide derivative is to be fixed. In cases where the functional group X is carboxylic group or thiol group, it is preferred to protect the functional group with an appropriate protection group known in the art until use.

In the formulae [I] and [II], Y represents a linker. Preferred linker may include $C_2$ - $C_{30}$ linear or branched chain groups such as linear or branched alkylene groups. The more the number of carbon atoms in the linker, the less the solubility in water or hydrophilicity of the oligonucleotide derivative. Thus, in the case where the linker Y is an alkylene group, the alkylene group may preferably have carbon atoms of 2 - 10. When it is desired that the oligonucleotide derivative have a linker Y longer $C_{10}$ while substantially not adversely affecting the high solubility in water or hydrophilicity of the oligonucleotide derivative, the linker Y may preferably contain one or more hydrophilic groups. Preferred examples of the hydrophilic groups may include secondary amino group, ether group, amide group, tertiary amino group, hydroxyl group, disulfide group and cyclohexyl group. The linker Y may also contain a functional group which can react with other substances, such as a branch having amino group. By incorporating one or more functional groups in the linker Y, an oligonucleotide derivative may be obtained which can be labelled with more than one markers and which can be well fixed to a carrier.

The oligonucleotide derivative of the present invention may be prepared starting from an oligonucleotide derivative represented by the above-described formula [III].

In formula [III], $B''$ and $B'''$ represent a base, that is, adenine, cytosine, guanine or thymine, of which amino group may be protected. The base sequence of the oligonucleotide derivative of the formula [III] corresponds to that of the oligonucleotide derivative of the formula [I] or [II]. The amino groups in the base $B'$ and $B'''$ may preferably be protected by a protection group which can be removed under alkaline

conditions. Preferred examples of such a protection group may include benzoyl group and isobutyryl group which can be removed by a treatment with 28 wt% aqueous ammonia at 55 °C for 5 - 24 hours.

In formula [III], $-COR^1$ is a protection group of the hydroxyl group of the 3′ end of the oligonucleotide. Protection groups which can be removed under alkaline conditions are preferred. Ester group or the like which can be removed under 28 wt% aqueous ammonia solution is a preferred example. $R^1$ may preferably be a lower alkyl group or aryl group or a carrier such as silica having an appropriate spacer which is usually used in solid phase synthesis. To simplify the operation, a synthetic carrier such as silica (controlled pored glass) having an appropriate spacer such as the following may preferably be employed as the group $R^1$.

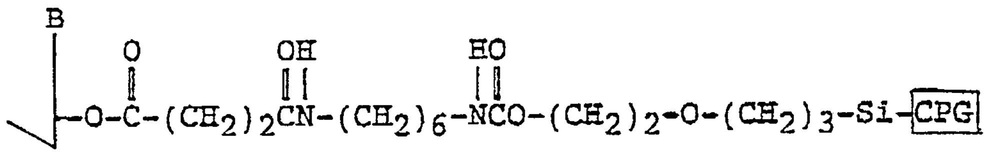

In formula [III], $R^2$ is a protection group of the phosphate group. Protection groups which can be removed by treatment with thiophenol or under alkaline conditions are preferred. Examples of such protection groups include methyl group, *β*-cyanoethyl group and the like.

The oligonucleotide derivative of the formula [III] may be prepared by a known method such as triester method or phosphoamidite method (*Synthesis of Nucleoside and Nucleotide*, Maruzen Publishing Co.,), although the preparation method is not restricted thereto.

The oligonucleotide derivative of the formula [III] is reacted with N,N′-carbonyldiimidazole to form an active intermediate represented by the above-described formula [IV]. This reaction may be carried out in an organic solvent such as acetonitrile or acetone, at a temperature of, for example, 20 to 30 °C for 0.1 to 1 hour.

Alternatively, the oligonucleotide derivative of the formula [III] is reacted with a 2-fluoro-1-methyl-pyridinium salt to form an active intermediate represented by the above-described formula [V]. This reaction may also be carried out in the same conditions for as the reaction for producing the active intermediate of the formula [IV].

The active intermediate [IV] or [V] is then reacted with a compound represented by the formula $NH_2$-Y-X (wherein Y and X represent the same meanings as in formula [I]) which is hereinafter referred to as "linker compound".

As mentioned above, the linker Y may be an alkylene group and X may be primary amine group. In this case, the linker compound is represented by the formula $NH_2-(CH)_n-NH_2$ (n represents a natural number). In the case of n = 1, the linker compound is diaminomethane. In the case of n = 2,3,4,5,6 •••, the linker compound is ethylenediamine, trimethylenediamine, tetramethylenediamine, pentamethylenediamine, hexamethylenediamine, •••. As mentioned earlier, since the hydrophobicity is increased with the increase in the number of carbon atoms in the linker Y, it is preferred to employ a linker compound having not more than 10 carbon atoms in cases where Y is an alkylene group. The linker compound may be linear or branched.

As mentioned above, by incorporating one or more hydrophilic groups in the linker compound, the length of the linker Y may be made longer without decreasing the hydrophilicity so much. For example, the linker compound may be one containing one or more $-CH_2-CH_2-NH-$ units. Examples of this type of compounds include tetraethylenepentamine, pentaethylenehexamine and the like. Alternatively, the linker compound may be one which contains one or more $-CH_2-CH_2-O-$ units. Examples of this type of compounds include bis (3-aminopropyl) ether, ethyleneglycol-bis(3-aminopropyl) ether, diethyleneglycol-bis-(3-aminopropyl) ether, 1,4-butandiol bis(3-aminopropyl) ether and the like. As mentioned above, linker compounds containing amido bond, tertiary amino group, hydroxyl group, disulfide group or cyclohexyl group may also preferably be employed. Further, the linker Y may contain a functional group such as amino group as mentioned earlier.

The reaction between the active intermediate of the formula [IV] or [V] and the linker compound may be carried out in an organic solvent such as acetonitrile or acetone at a temperature of, for example, 20 to 30 °C for 0.1 to 1 hour.

By this reaction, the oligonucleotide derivative of the present invention of the formula [I] or [II] may be obtained. More particularly, from the active intermediate of the formula [IV], the oligonucleotide derivative of the formula [I] is formed, and from the active intermediate of the formula [V], the oligonucleotide derivative of the formula [II] is formed.

The oligonucleotide derivative of the present invention may be used as a ligand for affinity chromatography for separating a nucleic acid having the base sequence complementary to the base sequence of the oligonucleotide derivative. That is, the oligonucleotide derivative of the present invention may be fixed to a chromatography packing such as silica gel through the functional group X. Alternatively, the oligonucleotide derivative of the present invention may be used as a DNA probe after labelling the oligonucleotide derivative with an appropriate marker. Further, the oligonucleotide derivative of the present invention may be used as a primer used in the determinations of base sequences of nucleic acids such as Sanger's method.

The present invention will now be described by way of preferred examples thereof. The examples are presented for are illustration purpose only and should not be interpreted in any restrictive way.

## Example 1

An oligonucleotide represented by the formula [III] wherein m is 20, $R^1$ is a glass bead having a spacer, $R^2$ is $\beta$-cyanoethyl group, the protection group of the amino groups in $B''$ and $B'''$ is benzoyl group in the case of adenine and cytosine and isobutyryl group in the case of guanine was prepared by a DNA synthesizer (381A commercially available from Applied Biosystems). The DNA synthesizer is a machine which synthesizes DNAs by phosphoamidite method. The base sequence of the oligonucleotide derivative was as follows:

5′-CCAGTCACGACGTTGTAAAC-3′

The oligonucleotide derivative having a hydroxyl group at its 5′ end was reacted with 0.5 M N,N′-carbonyldiimidazole in acetonitrile at room temperature for 15 minutes and then with 1.0 M ethylenediamine in acetonitrile at room temperature for 15 minutes. The oligonucleotide derivative was then treated with 28 wt% of aqueous ammonia at room temperature for 11 hours to liberate the oligonucleotide derivative from the solid phase. The liberated oligonucleotide derivative was heat-treated *in situ* at 55 °C for 17 hours so as to remove the protection groups.

## Example 2

The oligonucleotide derivative obtained in Example 1 was subjected to ion-exchange chromatography. That is, the oligonucleotide derivative obtained in Example 1 was applied to a column containing an ion-exchange resin having DEAE groups (DEAE-NPR column, commercially available from TOSOH CORPORATION) equilibrated with 20 mM Tris-HCl buffer (pH 9.0) and was then eluted by NaCl gradient of 0.2 - 0.5 M in 20 mM Tris-HCl buffer (pH 9.0) at a flow rate of 1.0 ml/min. As a control, the oligonucleotide derivative of the formula [III] employed as the starting material in Example 1 was also subjected to the same chromatography.

As a result, the oligonucleotide derivative having a hydroxyl group at the 5′ end, that is, the starting material was eluted at an NaCl concentration of about 0.33M. On the other hand, the oligonucleotide derivative of the present invention prepared in Example 1 was eluted at an NaCl concentration of about 0.32M. These results indicate that oligonucleotide derivative of which 5′ end was aminated was obtained in Example 1.

## Example 3

The same procedure as in Example 2 was repeated except that the ion-exchange resin column was DEAE-5PW column commercially available from Tosoh Corporation, and the buffer used for equilibrating the column and for the elution was 20 mM phosphate buffer (pH 6.8).

As a result, the oligonucleotide derivative having a hydroxyl group at the 5′ end was eluted at an NaCl concentration of about 0.4 M and the oligonucleotide derivative of the present invention obtained in Example 1 was eluted at an NaCl concentration of about 0.38 M. These results indicate that oligonucleotide derivative of which 5′ end was aminated was obtained in Example 1. The chromatogram is shown in Fig. 1.

## Example 4

Three hundred micrograms of the oligonucleotide derivative prepared in Example 1 was dissolved in 50

μl of 1M carbonic acid buffer (pH 9.0), and 50 μl of fluorescein isothiocyanate in dimethylformamide FITC (10mg/ml) was added to the solution. The resulting mixture was left to stand overnight at room temperature. The reaction product was separated by gel permeation chromatography (Sephadex G-25, commercially available from Pharmacia). After condensation, the resultant was subjected to reverse phase chromatography column (ODS-120T, commercially available from Tosoh Corporation) and peak fractions obtained at a retention time of 23 - 24 minutes were recovered. The reverse phase chromatogram is shown in Fig. 2.

The recovered peak fractions were analyzed for their absorption spectrum at 220 - 600 nm. From the absorbance at 260 nm which is the absorption peak of nucleic acids, and from the absorbance at 490 nm which is the absorption peak of the fluorescein, the ratio of the molecular weight was calculated utilizing the molar absorption coefficients. As a result, the ratio of the nucleic acid to the fluorescein was 1.012.

Since one fluorescein molecule binds only to one amino group in the oligonucleotide derivative, the oligonucleotide derivative obtained in Example 1 has one amino group at its 5′ end.

## Example 5

The oligonucleotide derivative of the formula [III] employed in Example 1 was prepared by the DNA synthesizer, and was reacted with 0.5 M N,N′-carbonyldiimidazole in acetonitrile so as to form an active intermediate. The active intermediate was reacted with 1.0 M methyl 6-amino-1-caproate hydrochloric acid salt in (acetonitrile/triethylamine) at room temperature for 30 minutes.

It should be noted that methyl 6-amino-1-caproate hydrochloric acid salt was prepared by activating methanol with thionyl chloride and by subsequent reaction with 6-amino-1-caproic acid. Triethylamine was used for the purpose on dissolving methyl 6-amino-1-caproate hydrochloric acid salt in acetonitrile.

The oligonucleotide derivative was liberated from the solid phase and was subjected to deprotection as in Example 1.

## Example 6

The oligonucleotide derivative of prepared in Example 5 and the oligonucleotide derivative prepared by the DNA synthesizer were subjected to ion-exchange chromatography as in Example 2. As a result, the oligonucleotide derivative synthesized by the DNA synthesizer was eluted at an NaCl concentration of about 0.33 M, while the oligonucleotide derivative of the present invention prepared in Example 5 was eluted at an NaCl concentration of about 0.34 M. These results indicate that the oligonucleotide derivative of the present invention prepared in Example 5 has carboxyl group at its 5′ end.

## Example 7

The oligonucleotide derivative of the formula [III] employed in Example 1 but having a base sequence of 5′-GTTGGGGACTGCGAATTTTGG-3′ was prepared by the DNA synthesizer, and was reacted with 0.5 M N,N′- carbonyldiimidazole in acetonitrile so as to form an active intermediate. The active intermediate was reacted with 1.0 M pentaethylenehexamine in acetonitrile, 1.0 M N,N-bis(3-aminopropyl)methyleneamine in acetonitrile, or 1.0 M ethyleneglycol-bis(3-aminopropyl) ether in acetonitrile.

The oligonucleotide derivative was liberated from the solid phase and was subjected to deprotection as in Example 1.

## Example 8

The oligonucleotide derivative of the present invention prepared in Example 7 and the oligonucleotide derivative synthesized by the DNA synthesizer in Example 7 were subjected to the ion-exchange chromatography in the same manner as in Example 3.

As a result, the oligonucleotide derivative prepared by using pentaethylenehexamine, which has $-CH_2-CH_2-NH-$moiety in the linker Y was eluted at an NaCl concentration of about 0.365 M. The oligonucleotide derivative prepared by using N,N-bis(3-aminopropyl)methylamine, which has a tertiary amine in the linker Y was eluted at an NaCl concentration of about 0.375 M. The oligonucleotide derivative prepared by using diethyleneglycolbis(3-aminopropyl) ether, which has $-CH_2-CH_2-O-$ moiety in the linker Y was eluted at an

NaCl concentration of about 0.38 M. These results indicate that oligonucleotide derivative having different linkers exhibit different elution time from the column.

Example 9

In the same manner as in Example 1, thymidine 21-mer having a hydroxyl group at its 5′ end was prepared. The hydroxyl group at the 5′ end was converted to amino group in the same manner as in Example 1. The thus prepared oligonucleotide derivative or the oligonucleotide derivative having a hydroxyl group at the 5′ end was dissolved in 1 M phosphate buffer (pH 9.0), and 2 ml of each solution was added to 100 mg of a gel (Tresyl-NPR, commercially available from Tosoh Corporation). After allowing to react to room temperature for 24 hours, the mixture was centrifuged.

The amount of the DNA fixed to the gel was determined from the amount of the DNA contained in the 2 ml of the solution and the amount of the DNA remained in the supernatant after centrifugation based on a calibration curve of absorption at 260 nm prepared by using standard DNA solutions with known concentrations of the DNA. As a result, the amount of the fixed DNA was 0.26 µg/mg dry gel in the case of the thymidine 21-mer having a hydroxyl group at the 5′ end while that of the thymidine 21-mer having an amino group at the 5′ end was 2.73 µg/mg dry gel, which is as much as about 10 times the amount of the thymidine 21-mer having hydroxyl group at its 5′ end.

## Claims

1. An oligonucleotide derivative represented by the formula [I]:

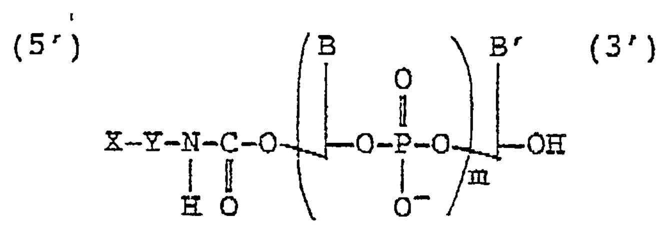

[I]

(wherein m represents a natural number, B and B′, the same or different, represent a base selected from the group consisting of adenine, thymine, cytosine and guanine,

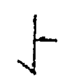

represents a deoxyribose residue which lacks hydroxyl groups on 3′ and 5′ position of 2′-deoxyribose, X represents a functional group which can react with other substances, and Y represents a linker).

2. An oligonucleotide derivative represented by the formula [II]:

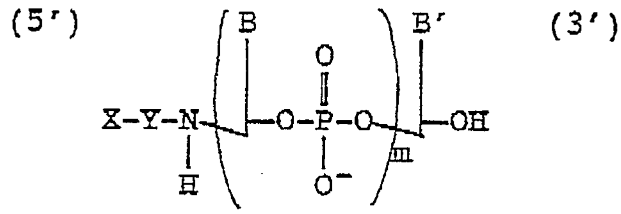

[II]

(wherein m, B, B′, X,

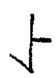 ,

and Y represent the same meanings as in formula [I]).

3. The oligonucleotide derivative of claim 1, wherein Y represents a linear or branched $C_2$ - $C_{30}$ chain group.

4. The oligonucleotide derivative of claim 2, wherein Y represents a linear or branched $C_2$ - $C_{30}$ chain group.

5. A process of producing the oligonucleotide derivative of claim 1, comprising the steps of:

reacting an oligonucleotide derivative represented by the formula [III]:

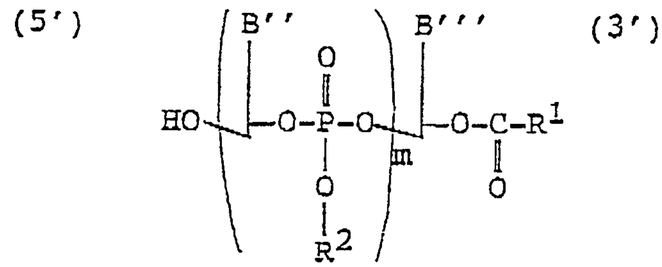

[III]

(wherein $-COR^1$ represents a protection group of the hydroxyl group at the 3′ end of the oligonucleotide derivative, $R^2$ represents a protection group of the phosphate group, m and

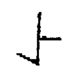

represent the same meanings as in formula [I], B″ and B‴, the same or different, represent adenine, cytosine, guanine or thymine of which amino group may be protected)

with N,N′-carbonyldiimidazole to form an active intermediate represented by the formula [IV]:

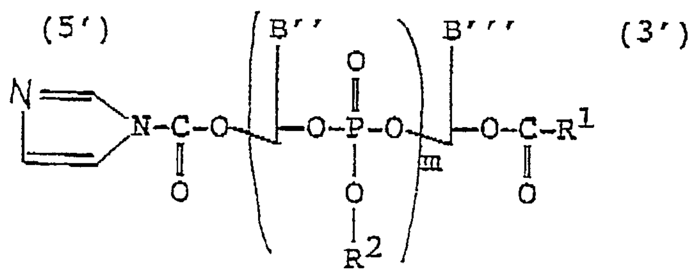

[IV]

(wherein m, B″, B‴,

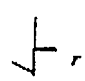

$R^1$ and $R^2$ represent the same meanings as in formula [III]);

reacting the obtained active intermediate with a compound represented by the formula $NH_2$-Y-X (wherein Y and X represent the same meanings as in formula [I]); and

removing said protection groups represented by $-COR^1$ and $-R^2$.

6. A process of producing the oligonucleotide derivative of claim 2, comprising the steps of:

reacting said oligonucleotide derivative represented by said formula [III] with a 2-fluoro-1-methylpyridinium salt to form an active intermediate represented by the formula [V]:

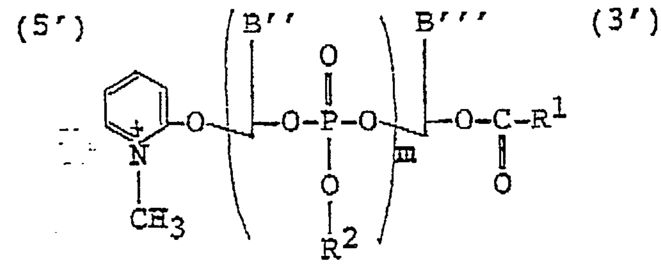

[V]

(wherein m, B″, B‴,

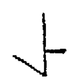 ,

$R^1$ and $R^2$ represent the same meanings as in formula [III]);
reacting the obtained active intermediate with a compound represented by the formula $NH_2$-Y-X (wherein Y and X represent the same meanings as in formula [I]); and
removing said protection groups represented by $-COR^1$ and $-R^2$.

7. An oligonucleotide derivative as claimed in claims 1 to 4 wherein m is a number from 5 to 100.

8. An oligonucleotide derivative as claimed in claims 1 to 4 and 7 wherein X represents a primary or secondary amino acid, a carboxylic acid group, a thiol group or a hydroxyl group.

9. An oligonucleotide derivative according to claims 3 or 4 wherein Y contains one or more hydrophilic groups, for example secondary or tertiary amino, ether, hydroxyl disulphide and cyclohexyl.

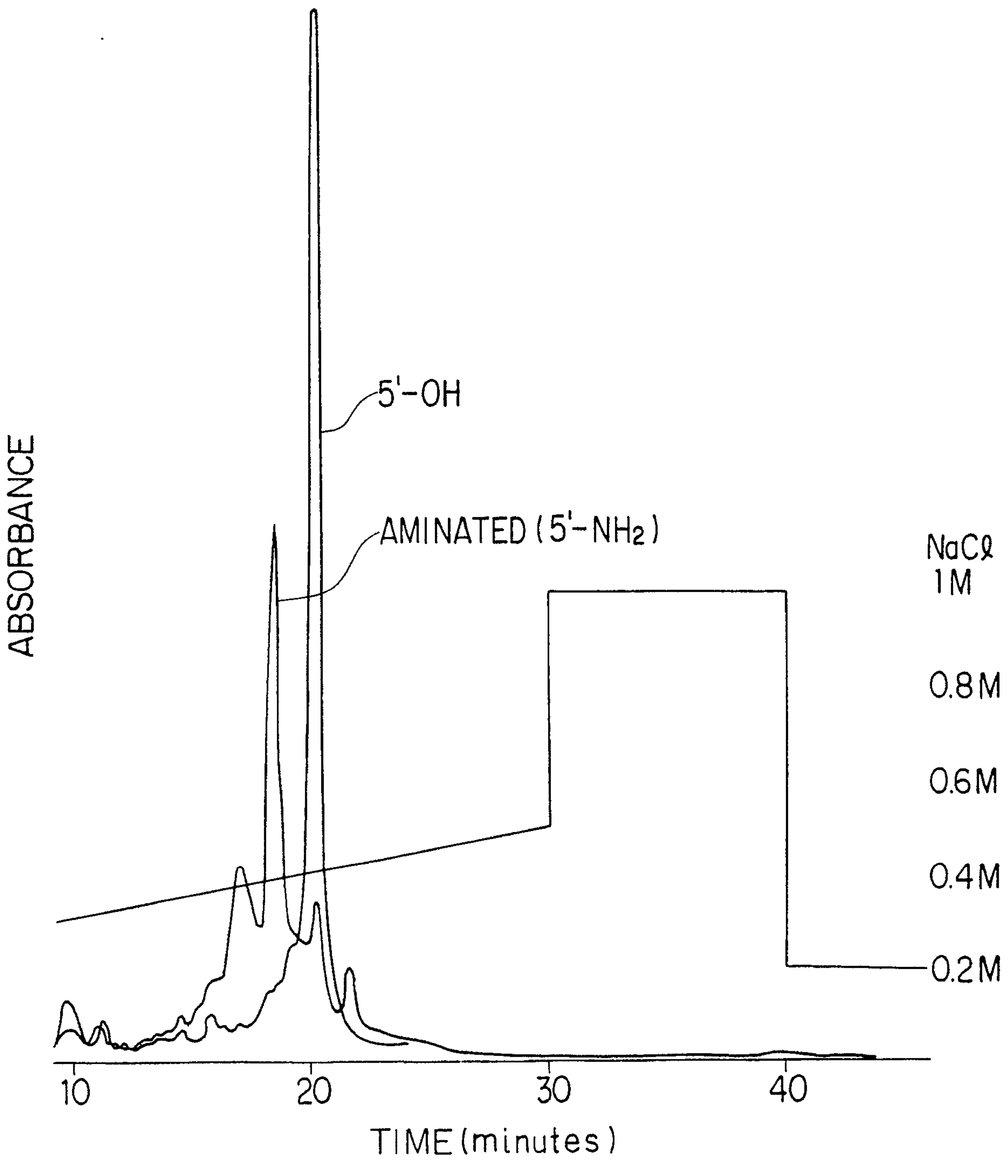
5'-OH
ABSORBANCE
AMINATED (5'-NH2)
NaCl
1M
0.8M
0.6M
0.4M
0.2M
10
20
30
40
TIME (minutes)

F I G. 1

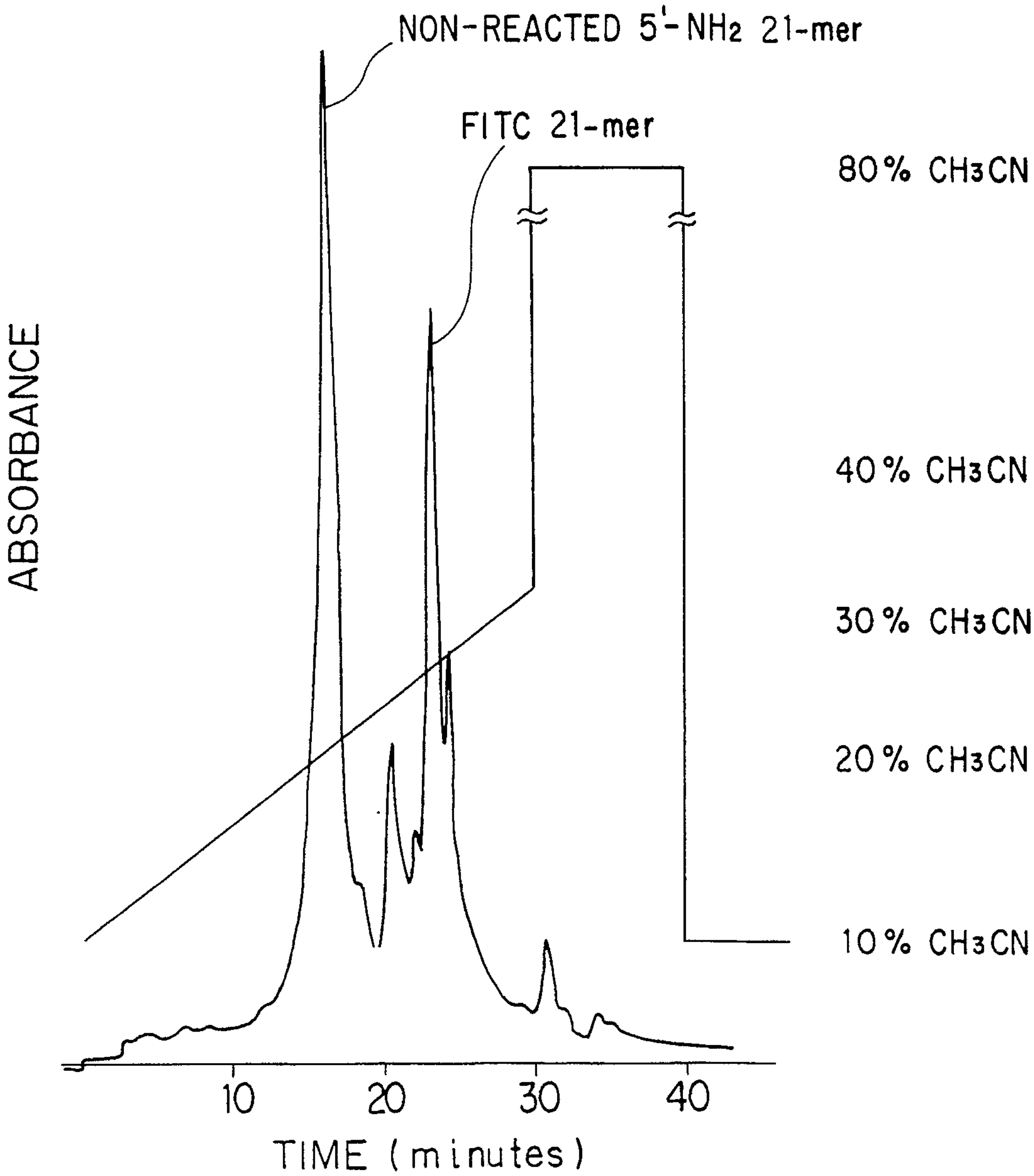
NON-REACTED 5'-NH2 21-mer
FITC 21-mer
80% CH3CN
40% CH3CN
30% CH3CN
20% CH3CN
10% CH3CN
ABSORBANCE
10
20
30
40
TIME (minutes)

FIG. 2